# EUROPEAN PATENT APPLICATION

(11) **EP 4 599 835 A1**
(43) Date of publication of application: **13.08.2025**
(21) Application number: 23888597.4
(22) Date of filing: 01.11.2023
(51) Int. Cl.: A61K 35/74, A01N 63/22, A01P 1/00, A61P 31/14, A61P 31/16, C12N 1/20

(54) **VIRAL INFECTION INHIBITOR**

(30) Priority: 07.11.2022 JP 2022178336
(71) Applicant: Japan Eco-Science Co. Ltd., Chiba-shi, Chiba, 263-8522 (JP); Miroku Co. Ltd., Oita 873-0021 (JP); Keiyo Gas Energy Solution Co., Ltd., Chiba 272-0015 (JP); Sermas Co., Ltd., Ichikawa-shi, Chiba 272-0015 (JP)
(72) Inventor: MIYAMOTO Hirokuni, Chiba-shi, Chiba 263-8522 (JP); MIYAMOTO Hisashi, Kitsuki-shi, Oita 873-0021 (JP); YAMAMOTO Naoki, Chiba-shi, Chiba 263-8522 (JP)
(74) Representative: Berggren Oy
(86) International application number: PCT/JP2023/039494
(87) International publication number: WO 2024/101249

(57) **Abstract**

The present invention addresses the problem of providing a novel, environmentally friendly infection inhibitor for infectious disease viruses (RNA viruses, DNA viruses). The present invention provides a viral infection inhibitor that includes microorganisms of genus Caldibacillus and genus Paenibacillus.

## Description

### TECHNICAL FIELD

The present invention relates to a virus infection inhibitor.

### BACKGROUND ART

Viruses are protein structures that contain nucleic acids capable of autonomously replicating by utilizing host cells. From these characteristics, it has been long argued whether viruses are living organisms or non-living organisms. If viruses are regarded as living organisms, it is predicted that viruses are the most abundant on the earth and there are 10³¹ of them (Non-Patent Document 1). Viruses historically play a wide range of roles in ecosystems, and play an important role in the evolutionary process of animals. Recognizable evidence thereof is a gene essential for an important process during pregnancy, placentation, that is integrated in the human genome (an example of exaptation, Non-Patent Document 2). In a broader perspective, it is reported that viruses affect the ecological balance and are involved in suppression of outbreak of red tide in the sea (Non-Patent Document 3).

As discussed above, viruses thus widely affect physiological functions of animals and plants and ecosystems. However, an aspect thereof as a pathogen is the commonly known function thereof. Spread of infection with virulent viruses may cause enormous damage and serious social problems. Such pathogenic viruses include coronavirus, influenza virus and swine fever virus. All of these viruses are categorized as enveloped RNA viruses and are known as causative viruses of human or animal infections.

Various coronaviruses are known and include porcine epidemic diarrhea (PED) virus that belongs to the Alphacoronavirus and cause acute diarrhea in pigs; SARS coronavirus 2 (SARS-CoV-2) that belongs to the Betacoronavirus and caused the recent worldwide pandemic of novel coronavirus infection (COVID-19); and avian infectious bronchitis virus (IB) that belongs to the Gammacoronavirus. In addition, seasonal influenza virus, avian influenza (H5N1) virus for which not only the risk thereof but also serious negative economic impact due to killing of the animals are concerned, and swine fever virus (Flaviviridae, Pestivirus) can be mentioned. These viruses, because of the characteristics of RNA viruses, have a rapid rate of genetic mutation and may generate various subtypes within a relatively short period of time, and hence are always accompanied by emergence of unknown mutants. Consequently, there may be different mutants in different regions, and thus there is a problem that vaccines effective against mutants cannot be prepared in time.

Due to this problem, it is urgently needed to develop techniques for inhibiting virus infection. For example, various techniques for preventing and inhibiting infection with coronavirus have been proposed (for example, Patent Documents 1 and 2).

However, conclusive techniques for inhibiting infection are mainly drug components that are artificially synthesized such as ethanol and benzalkonium chloride, and do not take into account conservation of ecosystems or interactions with symbiotic bacteria that live together with humans and animals. Because of the above, it is considered that the techniques may cause other adverse effects even if the techniques have an effect for inhibiting viral infection. Thus, as suggested in "Hygiene Hypothesis" proposed in 1989, extreme hygiene control in the modern society contributes to occurrence of various diseases (Non-Patent Document 4). Therefore, there is a need for achieving inhibition of infection with pathogenic viruses while properly maintaining symbiosis between humans and animals and microorganisms.

### Citation List

### Patent Document

Patent Document 1: Japanese Unexamined Patent Application, Publication No. 2000-044473
Patent Document 2: Japanese Unexamined Patent Application, Publication No. 2022-067656

### Non-Patent Document

Non-Patent Document 1: Translation supervised by Kunitada SHIMOTOHNO and Tsukasa SEYA, Virology for Life Science: Mechanism of Infection and Host Response Medical Applications Nankodo Co., Ltd.
Non-Patent Document 2: Hirokuni MIYAMOTO, "Chapter 22 Environmental Microorganisms and Animals", Hiroshi OHNO Ed., "Symbiotic Microorganisms", Dojin Bioscience series No. 27, pp. 247-256 (2016)
Non-Patent Document 3: Yuji TOMARU, Yoko SHIRAI, Yoshitake TAKAO and Keizo NAGASAKI, Featured Topic "Aquatic Biomass and Environmental Restoration", "Smallest Biotic Factors in the Sea Water - Ecology of Marine Viruses", Bull. Soc. Sea Water Sci., Jpn., 61, 307-315 (2007)
Non-Patent Document 4: Strachan DP (1989) Hay fever, hygiene, and household size. BMJ 299 (6710): 1259-1260

### DISCLOSURE OF THE INVENTION

### Problems to be Solved by the Invention

There is a need for a technique that does not cause adverse effects on ecosystems and microorganisms symbiotic with humans and animals like artificially synthesized drugs and has an effect for inhibiting infectivity against overall viruses having a high rate of mutagenesis and requiring a long time for developing vaccines therefor. In light of a further measure for preventing infection, such a technique is expected to not only cut off the source of infection but also allow operation for achieving a sustainable society.

In view of the foregoing, it is an object of the present invention to provide a novel, environmentally symbiotic infection inhibitor against infectious viruses (such as RNA viruses and DNA viruses).

### Means for Solving the Problems

The present inventors found that certain mixed microorganisms can solve the above problem, thereby completing the present invention. The present invention therefore specifically provides the following.
(1) A virus infection inhibitor, containing a microorganism of the genus Caldibacillus or a culture thereof.
(2) The virus infection inhibitor according to (1), in which the microorganism of the genus Caldibacillus contained is a microorganism having NITE Accession No. "BP-863".
(3) The virus infection inhibitor according to (1), in which the virus infection inhibitor is an environmentally symbiotic virus infection inhibitor containing mixed microorganisms having a symbiotic function with humans, animals and plants and an ecosystem, and in which the mixed microorganisms are of the genus Caldibacillus and the genus Paenibacillus.
(4) The environmentally symbiotic virus infection inhibitor according to (3), in which the mixed microorganisms contain a microorganism having NITE Accession No. "BP-03693".
(5) The environmentally symbiotic virus infection inhibitor according to (4), in which the mixed microorganisms containing NITE Accession No. "BP-03693" contain ATCC Accession No. "PTA-1773".
(6) The virus infection inhibitor according to (5), in which the mixed microorganisms having ATCC Accession No. "PTA-1773" contain a microorganism having NITE Accession No. "BP-863" and a microorganism having NITE Accession No. "BP-1051"
(7) The virus infection inhibitor according to any one of (1) to (6), in which the virus is porcine epidemic diarrhea virus belonging to Alphacoronavirus, SARS-CoV-2 belonging to Betacoronavirus or avian infectious bronchitis virus (IB) belonging to Gammacoronavirus.
(8) The virus infection inhibitor according to any one of (1) to (6), in which the virus is seasonal influenza virus, avian influenza (H5N1) virus or swine fever virus (Flaviviridae, Pestivirus).
(9) The virus infection inhibitor according to any one of (1) to (6) that does not have a negative impact on symbiotic organisms, in which the virus is norovirus, feline calicivirus, norovirus, Norwalk virus or rabbit hemorrhagic virus all of which are of the family Caliciviridae, a plant infectious virus (begomovirus, tobamovirus, nepovirus, tobravirus), a bacteriophage (Cystoviridae) or a hepatitis virus.

### Effects of the Invention

According to the present invention, a novel, environmentally symbiotic infection inhibitor against viruses (such as RNA viruses and DNA viruses) is provided.

### BRIEF DESCRIPTION OF THE DRAWINGS

Fig. 1 shows an effect of the infection inhibitor of the present invention against porcine epidemic diarrhea virus (PED virus P-5V strain);
Fig. 2 shows an effect of the infection inhibitor of the present invention against SARS-CoV-2 coronavirus (delta strain);
Fig. 3 shows an effect of the infection inhibitor of the present invention against influenza virus (swine influenza virus H1N1 IOWA strain);

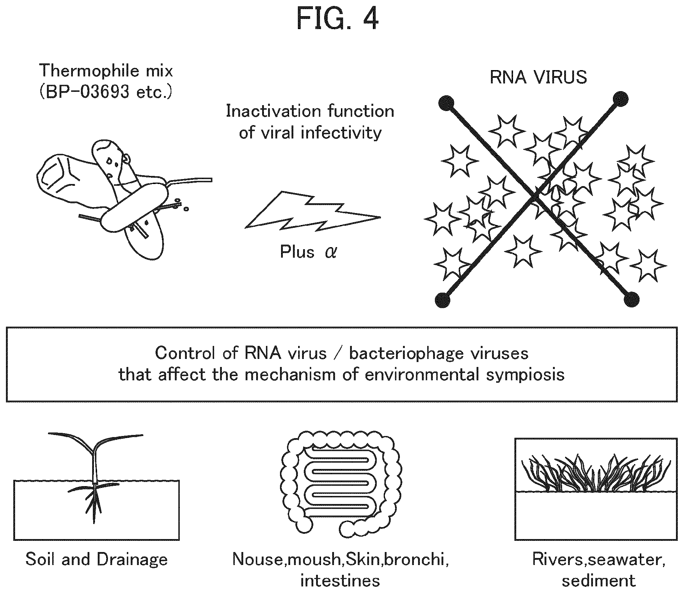

Fig. 4 illustrates examples of applications of the infection inhibitor of the present invention;

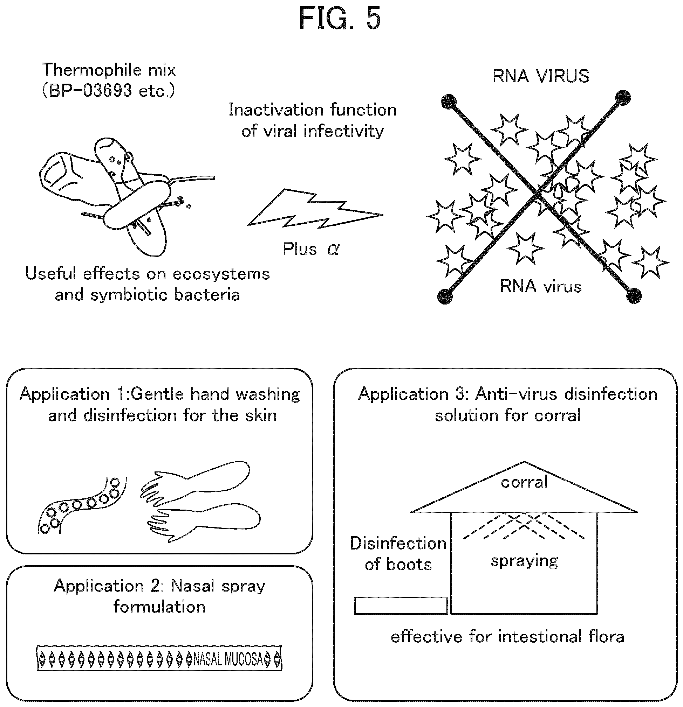

Fig. 5 illustrates examples of applications of the infection inhibitor of the present invention;
Fig. 6 illustrates an example of social implementation of the present invention; and
Fig. 7 illustrates an example of social implementation of the present invention.

### PREFERRED MODE FOR CARRYING OUT THE INVENTION

Embodiments of the present invention are hereinafter described in detail. It should be noted that the present invention is not limited to the embodiments described below.

### <Virus infection inhibitor>

The virus infection inhibitor (hereinafter also referred to as "infection inhibitor of the present invention") of the present invention contains a microorganism of the genus Caldibacillus or a culture thereof.

The infection inhibitor of the present invention preferably may contain one or more bacteria selected from the group consisting of ATCC accession No. "PTA-1773", NITE accession No. "BP-03693" and NITE accession No. "BP-863". The above bacteria include microorganisms of the genus Caldibacillus.

As used herein, the "culture of a microorganism" encompasses any culture obtained by culturing the microorganism in conditions (such as media and temperature) under which the microorganism can grow. Examples of a culture solution may include a culture solution (such as sterilized solution or non-sterilized solution) containing a microorganism and a culture supernatant that does not contain a microorganism. Examples of the media include animal or plant proteins.

### (1) ATCC accession No. "PTA-1773"

The mixed microorganisms "PTA-1773" are thermophilic seed bacteria deposited with "the American Type Culture Collection (ATCC)" (10801 University Boulevard Manassas, Virginia, 20110-2209, U.S.A.), which is an international depository authority under the Budapest Treaty on the international recognition of the deposit of microorganisms for the purposes of patent procedure on 10 May 2000. ATCC accession No. "PTA-1773" includes NITE accession Nos. "BP-863", "BP-1051" and "BP-03693".

"PTA-1773" includes microorganisms of the genus Caldibacillus and the genus Paenibacillus.

### (2) NITE accession No. "BP-03693"

The mixed microorganisms "BP-03693" are thermophilic seed bacteria deposited with "the National Institute of Technology and Evaluation" (2-5-8, Kazusakamatari, Kisarazu-shi, Chiba, Japan), which is an international depository authority under the Budapest Treaty on the international recognition of the deposit of microorganisms for the purposes of patent procedure on 27 July 2022. The mixed microorganisms "BP-03693" were separated from the mixed microorganisms "PTA-1773" and composed of three strains, Caldibacillus hisashii (NITE accession No. "BP-863", internationally deposited on 8 February 2010), and Paenibacillus strains Nos. 39 and 139. It was validated by "the National Institute of Technology and Evaluation" (2-5-8, Kazusakamatari, Kisarazu-shi, Chiba, Japan) on 31 October 2022 that "BP-03693" is composed of three strains, Caldibacillus hisashii, and Paenibacillus strains Nos. 39 and 139.

While "BP-863" was internationally deposited at first as a related species of Bacillus thermoamylovans (15 January 2010), "BP-863" was afterwards registered as a novel species of Bacillus hisashii (Nishida, A. et al. Bacillus hisashii sp. nov., isolated from the caeca of gnotobiotic mice fed with thermophile-fermented compost. Int J Syst Evol Microbiol 65, 3944-3949, doi:10.1099/ijsem.0.000516 (2015)). Due to the changes in the international microorganism classification, "BP-863" was further reclassified as Caldibacillus hisashii (Gupta, R. S., Patel, S., Saini, N. & Chen, S. Robust demarcation of 17 distinct Bacillus species clades, proposed as novel Bacillaceae genera, by phylogenomics and comparative genomic analyses: description of Robertmurraya kyonggiensis sp. nov. and proposal for an emended genus Bacillus limiting it only to the members of the Subtilis and Cereus clades of species. Int J Syst Evol Microbiol 70, 5753-5798, doi:10.1099/ijsem.0.004475 (2020)). As described in Gupta et al., according to the above classification changes, it was decided that conventional Bacillus thermoamylovorans and Bacillus hisashii are closely related and are both classified into the genus Caldibacillus. It was also validated by "the National Institute of Technology and Evaluation" (2-5-8, Kazusakamatari, Kisarazu-shi, Chiba, Japan) on 31 October 2022 that "BP-863" is classified into Caldibacillus hisashii.

"BP-03693" includes microorganisms of the genus Caldibacillus and the genus Paenibacillus.

### (3) NITE accession No. "BP-1051"

The mixed microorganisms "BP-1051" are thermophilic seed bacteria deposited with "the National Institute of Technology and Evaluation" (2-5-8, Kazusakamatari, Kisarazu-shi, Chiba, Japan), which is an international depository authority under the Budapest Treaty on the international recognition of the deposit of microorganisms for the purposes of patent procedure on 18 January 2011.

### (4) Effects and the like of the infection inhibitor of the present invention

The present inventors found in the previous research that "PTA-1773" and "BP-863" can activate the innate immune system of animals and further provide reduction in lipid accumulation by controlling the intestinal bacterial flora (WO 2011/099514). "PTA-1773" and "BP-1051" are also effective on plant growth and have a function of suppressing production of greenhouse gases from soil. As a result of further studies, the present inventors found that a fermented solution from mixed microorganisms containing "BP-03693" which is included in "PTA-1773" has a significant infection inhibitory effect against at least specific viruses (such as RNA viruses (such as coronavirus and influenza virus) and DNA virus (such as hepatitis B virus)).

As used therein, the term "mixed microorganisms" means a population of multiple types of microorganisms. As a result of genomic analysis of the deposited microorganisms described above, a potential presence of a phage-associated protein and a lantibiotic peptide was observed. Thus, these may be involved in viral infection inhibition.

As used herein, the "virus infection inhibitory effect" includes inhibition of viral replication in the presence of the infection inhibitor of the present invention. The virus infection inhibitory effect can be evaluated according to the methods described in the Examples.

The deposited microorganisms (particularly, those including microorganisms of the genus Caldibacillus and microorganisms of the genus Paenibacillus) described above are expected to have a symbiotic function with humans, animals and plants and an ecosystem and thus be environmentally symbiotic.

As used herein, the term "environmentally symbiotic" includes not compromising the balance of microflora such as skin indigenous bacteria or intestinal indigenous bacteria. For example, existing disinfectants (such as ethanol) tend to compromise the balance of the microflora and thus are hardly referred to as environmentally symbiotic.

As the infection inhibitor of the present invention is expected to be environmentally symbiotic, the infection inhibitor can reduce adverse effects on ecosystems and environments (such as soil, waste water, in vivo, rivers, sea water and bottom sediments) in addition to having an inhibitory function of virus infectivity.

As the infection inhibitor of the present invention is expected to be environmentally symbiotic, the infection inhibitor can realize disinfection on the skin, disinfection through the nose or disinfection of animal cages and the like with little impact.

### (5) Configuration and the like of the infection inhibitor of the present invention

The infection inhibitor of the present invention is not particularly limited except that the infection inhibitor may contain a microorganism of the genus Caldibacillus (such as "PTA-1773", "BP-03693", "BP-1051" or "BP-863") or a culture thereof. It should be noted that "PTA-1773" includes "BP-03693", "BP-1051" and "BP-863".

The contents of "PTA-1773", "BP-03693", "BP-1051" and "BP-863" may be set, as appropriate, according to the infection inhibitory effect to be achieved and the like. The contents (dry weight) of "PTA-1773", "BP-03693", "BP-1051" and "BP-863" may be, for example, respectively 0.001% by mass or more and 100% by mass or less relative to the infection inhibitor of the present invention.

The infection inhibitor of the present invention may contain any additive (such as microorganisms other than "PTA-1773", "BP-03693", "BP-1051" and "BP-863", a solvent (such as water), an excipient, a buffer and a preservative) within the range that does not adversely affect the action of "PTA-1773", "BP-03693", "BP-1051" and "BP-863". The type and amount of such an additive may be set, as appropriate, according to the effect to be achieved and the like.

In a preferable embodiment of the infection inhibitor of the present invention, the constituent microorganisms are only any of "PTA-1773", "BP-03693", "BP-1051" and "BP-863".

The infection inhibitor of the present invention may be in the form of liquid, solid or the like. The infection inhibitor of the present invention may be, for example, in the form that contains microorganism cells (such as a cell culture solution) or a culture supernatant of the microorganism (without containing cells).

The infection inhibitor of the present invention may be produced according to conventionally known manners according to the form thereof.

As "PTA-1773", "BP-03693", "BP-1051" and "BP-863" are all thermophilic, the microorganisms used may be sterilized when being added to the infection inhibitor of the present invention. By the sterilization, "PTA-1773", "BP-03693", "BP-1051" and "BP-863" can be added while preventing contamination of unwanted bacteria.

The sterilization temperature of "PTA-1773", "BP-03693", "BP-1051" and "BP-863" may be 40-100°C.

The sterilization period of "PTA-1773", "BP-03693", "BP-1051" and "BP-863" may be 1-120 minutes.

### (6) Viruses

Any virus without limitation may be a subject of inhibition of infection of the infection inhibitor of the present invention, and examples thereof include any coronaviruses that infect mammals (such as humans, pet animals (such as dogs and cats) and farm animals (such as pigs)) and mutants thereof.

The virus includes both RNA virus and DNA virus.

Examples of the RNA virus include porcine epidemic diarrhea virus belonging to the Alphacoronavirus, SARS-CoV-2 belonging to the Betacoronavirus and avian infectious bronchitis virus (IB) belonging to the Gammacoronavirus.

Examples of the RNA virus include seasonal influenza virus, avian influenza (H5N1) virus and swine fever virus (Flaviviridae, Pestivirus).

Examples of the RNA virus include plant viruses, bacteriophages, and feline calicivirus causing respiratory diseases in cats, norovirus causing infectious gastroenteritis in humans and the like, Norwalk virus and rabbit hemorrhage virus causing hemorrhage in rabbits that are non-enveloped RNA viruses of Caliciviridae.

Examples of the coronavirus include coronaviruses belonging to the subfamily Orthocoronavirinae. These coronaviruses can be classified into four genera (Alphacoronavirus, Betacoronavirus, Gammacoronavirus and Deltacoronavirus).

Examples of the coronavirus belonging to the Alphacoronavirus include porcine epidemic diarrhea virus and human coronavirus 229E.

Examples of the coronavirus belonging to the Betacoronavirus include SARS-associated coronaviruses (such as SARS coronavirus 2 (SARS-CoV-2)), betacoronavirus 1 and murine coronavirus.

Examples of the coronavirus belonging to the Gammacoronavirus include avian infectious bronchitis virus.

Examples of the coronavirus belonging to the Deltacoronavirus include bulbul coronavirus HKU11.

Examples of the influenza virus include swine influenza (H1N1) virus, seasonal influenza and avian influenza (H5N1) virus.

Examples of the swine fever virus include swine fever virus of Flaviviridae, Pestivirus.

Examples of the DNA virus include hepatitis B virus (HBV).

In light of readily obtaining the effects of the present invention, the infection inhibitor of the present invention preferably targets porcine epidemic diarrhea virus, SARS coronavirus 2 (SARS-CoV-2), swine influenza virus, hepatitis B (HBV) and the like. Examples of other preferable viruses include norovirus, feline calicivirus, norovirus, Norwalk virus and rabbit hemorrhagic virus all of which are of the family Caliciviridae, a plant infectious virus (begomovirus, tobamovirus, nepovirus, tobravirus), a bacteriophage (Cystoviridae) and a hepatitis virus.

### (4) Method for inhibiting virus infection using the infection inhibitor of the present invention

Infection with a virus can be inhibited by bringing the virus into contact with the infection inhibitor of the present invention by any arbitrary manner.

Any manner can be used without particular limitation for bringing the virus into contact with the infection inhibitor of the present invention. Examples of the manner include a manner in which the infection inhibitor of the present invention is sprayed towards a subject suspected to have a virus, nose drops and a manner in which the subject is wiped with an object impregnated with the infection inhibitor (such as a tissue paper soaked in the infection inhibitor of the present invention).

The subject with which the infection inhibitor of the present invention is brought into contact includes not only living organisms (such as the skin surface and inside the nostril) but also various articles that may come into contact with living organisms such as household items (such as surfaces of furniture, electric appliances and miscellaneous goods) and facilities (such as surfaces of fences and breeding facilities such as cages).

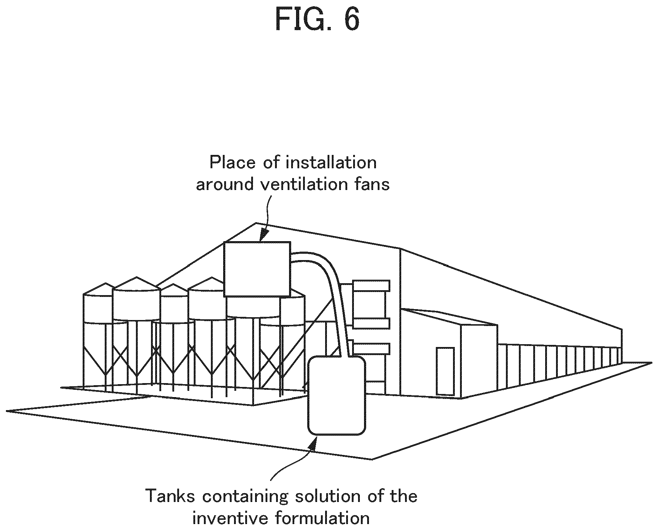

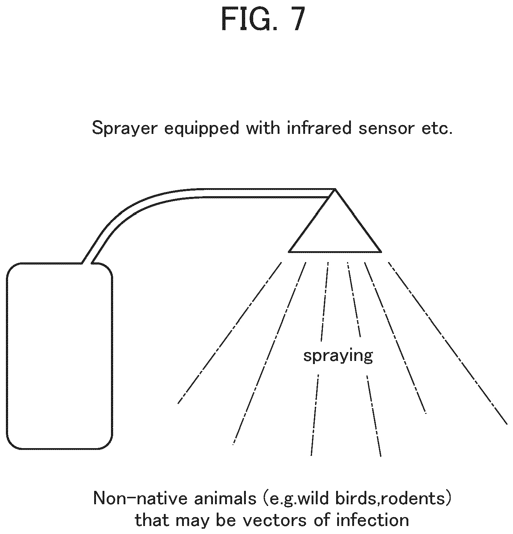

Figs. 6 and 7 show examples of social implementation of the present invention. As illustrated in Fig. 6, the infection inhibitor of the present invention may be placed in a container such as a tank which may be installed permanently to various facilities. In such an aspect, the infection inhibitor of the present invention can be sprayed, as illustrated in Fig. 7, to a non-native animal when the non-native animal comes into the facilities and is detected with an infrared sensor or the like. As a result, the source of infection such as a virus is less likely to enter the facilities. The infection inhibitor of the present invention hardly adversely affects intestinal bacteria unlike conventional disinfectants. Thus, the present invention leads to conservation in the environmentally symbiotic system and therefore meets the view (Nature positive) of biodiversity conservation that is sought worldwide.

### EXAMPLES

The present invention is hereinafter more specifically described on the basis of the Examples. However, the present invention is not limited by the Examples.

### <Test 1: Preparation of virus (RNA virus) infection inhibitor>

A sample was prepared according to the following manner using mixed microorganisms.

The mixed microorganisms of ATCC accession No. "PTA-1773" were 100-fold diluted with water and heated at 50°C for 1 hour or more. After the heating, the diluted microorganisms were filtered through a filter with a pore diameter of 100 µm to remove foreign substances, and the resulting filtrate was used as a sample (corresponding to the infection inhibitor of the present invention) and subjected to the following tests.

### <Test 2: Test for virus (RNA virus) infection inhibitory effect-1>

The sample obtained in "Test 1" was used to study the infection inhibitory effect of an RNA virus. The following test was carried out at Shokukanken Inc.

### (1) Preparation of virus and cultured cells

Porcine epidemic diarrhea virus (PEDV) P-5V strain, which is a pig infectious coronavirus, was prepared. Cultured cells used were Vero cells (cell strain derived from the kidney epithelium of African green monkey).

### (2) Virus inoculation

The sample was brought into contact with the virus on the basis of the following manner. The following test was carried out by referring to "Virus Experiments, Overview, 2nd revision, Maruzen Publishing Co., Ltd, Virus neutralization test method".

### (2-1) Preliminary test

Before the main test, an effect (cytotoxicity) of the sample on cultured cells was observed. First, the sample was diluted in a 10-fold series with a phosphate buffer and then inoculated to cultured cells, and the maximum concentration at which the cells after culturing showed normal state was confirmed and the virus concentration used for the test was decided. As a result, no cytotoxicity was observed in the 10-fold dilution. Thus, the detection limit of the main test was set to be 10^{1.5} TCID₅₀/mL.

### (2-2) Main test

According to the plots for the test indicated in Table 1, the sample and the phosphate buffer (10 mL each) were aliquoted and the virus solution (1 mL) at the concentration decided in the preliminary test was added. After the addition of the virus solution, the obtained test solutions were left at room temperature (25°C) for three hours.

**[Table 1]**

| Name of plot for test | Treatment | Sensitization period |
|---|---|---|
| Control plot | Mixing phosphate buffer with virus solution | Zero or 3 hours from addition of virus |
| Test plot (carried out twice) | Mixing sample with virus solution | Three hours from addition of virus |

The test solutions after the reaction were respectively diluted in a 10-fold series and inoculated to cultured cells in a 96-well plate at 100 µl. After the inoculation, the cells were cultured at 37°C for 5 days with carbon dioxide gas (5%). The obtained cultured cells were observed under a microscope, the presence or absence of virus replication was observed on the basis of CPE (cytopathogenicity) appeared on the cultured cells, and the concentration thereof was calculated for the respective plots.

On the basis of the obtained values, the rate (%) of virus reduction of the test plot relative to the control plot was calculated from the following equation. Rate (%) of virus reduction = (Control plot - Test plot)/Control plot × 100

On the basis of the above results, the rate (%) of reduction of the test plot relative to the control plot at 3 hours after the start of sensitization was calculated and the infection inhibitory effect was assessed. The rate of reduction was calculated according to the following equations: Logarithmic decrement = Log10(Viral titer of the control plot) - Log10(Viral titer of the test plot) Rate (%) of reduction = (1 - 1/10Logarithmic decrement) × 100

### (3) Results

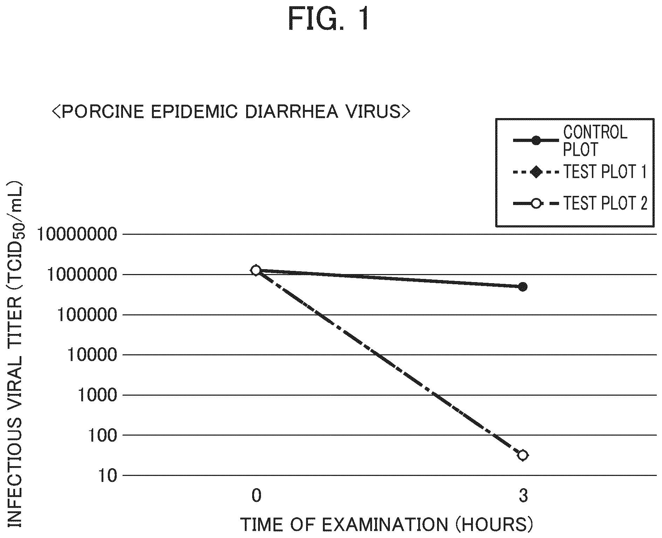

The results are illustrated in Fig. 1 and Table 2. In the control plot, a natural attenuation of the viral load was observed between the start of the test (at the time point when the virus was added) and three hours after the start of the test (10^{6.1} to 10^{5.7} TCID₅₀/mL). Meanwhile, in the test plot, a significant reduction of the viral load was observed at three hours after the start of the test (<10^{1.5} TCID₅₀/mL, rate of reduction=99.99% or more). Therefore, it was found that the thermophile fermentation solution which is the sample obtained in "Test 1" has such a high inactivation effect as 99.99% in the reaction of 180 minutes against porcine epidemic diarrhea virus. From the above results, it was found that the infection inhibitor of the present invention has an excellent infection inhibitory effect against porcine epidemic diarrhea virus.

**[Table 2]**

| Name of plot for test | Viral titer at the start of the test | Viral titer at three hours after the start of the sensitization | Rate of reduction (%) |
|---|---|---|---|
| Control plot | 10^{6.1} | 10^{5.7} | Natural attenuation |
| Test plot | 10^{6.1} | 10^{1.5} | 99.99 |

### <Test 3: Test for virus (RNA virus) infection inhibitory effect-2>

The sample obtained in "Test 1" was used to study the coronavirus infection inhibitory effect. The following test was carried out at Shokukanken Inc.

### (1) Test method

The test was carried out under the same conditions as "Test 2" described above except that SARS-CoV-2 coronavirus (delta strain) was used instead of the PEDV. The strain used in the present Example is a human-derived isolate and was confirmed for, after isolation and culture from saliva using Vero cells, amplification of SARS coronavirus 2 genes (Notification issued by the Ministry of Health, Labor and Welfare) and modification of N501Y (-) and L452R (+) by realtime PCR.

### (2) Results

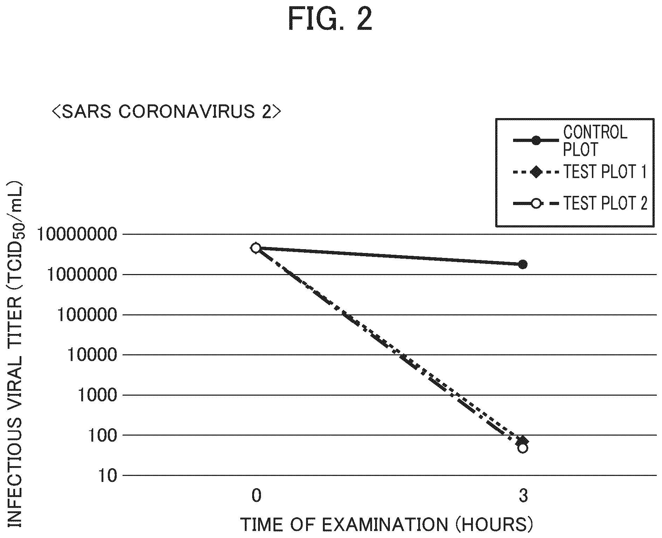

The results are illustrated in Fig. 2 and Table 3. In the control plot, a natural attenuation of the viral load was observed between the start of the test (at the time point when the virus was added) and three hours after the start of the test (10^{6.7} to 10^{6.3} TCID₅₀/mL). Meanwhile, in the test plot, a significant reduction of the viral load was observed at three hours after the start of the test (<10^{1.9} TCID₅₀/mL or 10^{1.7} TCID₅₀/mL, rate of reduction=99.99% in both cases). From the above results, it was found that the infection inhibitor of the present invention has an excellent infection inhibitory effect against SARS coronavirus 2.

**[Table 3]**

| Name of plot for test | Viral titer at the start of the test | Viral titer at three hours after the start of the sensitization | Rate of reduction (%) |
|---|---|---|---|
| Control plot | 10^{6.7} | 10^{6.3} | Natural attenuation |
| Test plot 1 | 10^{6.7} | 10^{1.9} | 99.99 |
| Test plot 2 | 10^{6.7} | 10^{1.7} | 99.99 |

### <Test 4: Test for virus (RNA virus) infection inhibitory effect-3>

The sample obtained in "Test 1" was used to study the swine influenza virus infection inhibitory effect. The following test was carried out at Shokukanken Inc.

### (1) Test method

The test was carried out under the same conditions as "Test 2" described above except that swine influenza virus H1N1 IOWA strain was used instead of the PEDV and MDCK cells (canine kidney-derived cell strain) were used as the cultured cells.

### (2) Results

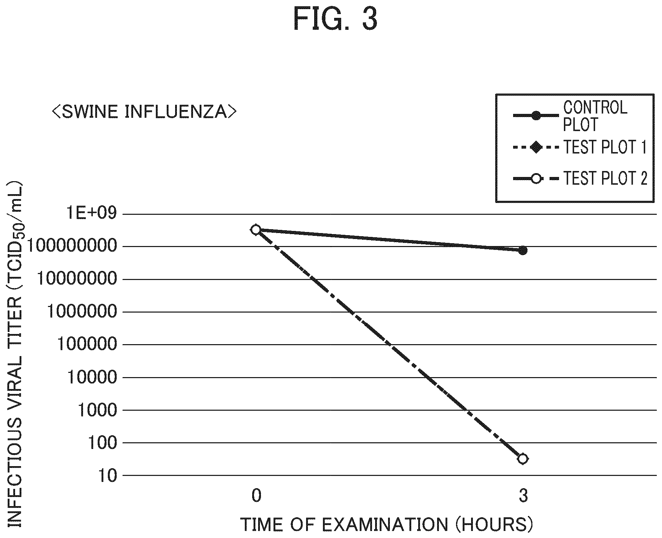

The results are illustrated in Fig. 3 and Table 4. In the control plot, a natural attenuation of the viral load was observed between the start of the test (at the time point when the virus was added) and three hours after the start of the test (10^{8.5} to 10^{7.9} TCID₅₀/mL). Meanwhile, in the test plot, a significant reduction of the viral load was observed at three hours after the start of the test (<10^{1.5} TCID₅₀/mL, rate of reduction=99.99% or more). From the above results, it was found that the infection inhibitor of the present invention has an excellent infection inhibitory effect against swine influenza virus.

**[Table 4]**

| Name of plot for test | Viral titer at the start of the test | Viral titer at three hours after the start of the sensitization | Rate of reduction (%) |
|---|---|---|---|
| Control plot | 10^{8.5} | 10^{6.3} | Natural attenuation |
| Test plot | 10^{8.5} | 10^{1.5} | 99.99 |

### <Test 5: Test for virus (RNA virus) infection inhibitory effect-4>

In addition to the above tests, an infection inhibitory effect against feline calicivirus which is a non-enveloped RNA virus of Caliciviridae causing respiratory diseases in cats was assessed.

The results are shown in Table 5. In the control plot, a natural attenuation of the viral load was observed between the start of the test and three hours after the start of the test (10^{8.5} to 10^{7.9} TCID₅₀/mL). In the test plot, the result was 10^{4.3} TCID₅₀/mL at three hours after the start of the test (rate of reduction: 99.97%).

**[Table 5]**

| Name of plot for test | Viral titer at the start of the test | Viral titer at three hours after the start of the sensitization | Rate of reduction (%) |
|---|---|---|---|
| Control plot | 10^{8.5} | 10^{7.9} | Natural attenuation |
| Test plot | 10^{8.5} | 10^{4.3} | 99.97 |

### <Test 6: Test for virus (RNA virus) infection inhibitory effect-5>

The sample obtained in "Test 1" was used to study the avian infectious bronchitis virus infection inhibitory effect. The following test was carried out at Shokukanken Inc.

### (1) Preparation of virus and cultured cells

Avian infectious bronchitis virus (IBV) H120 strain (Poulvac IB H120, Kyoritsu Seiyaku Corporation) was prepared as an avian infectious virus.

### (2) Virus inoculation

The sample was brought into contact with the virus on the basis of the following manner. The following test was carried out by referring to "Virus Experiments, Overview, 2nd revision, Maruzen Publishing Co., Ltd, Virus neutralization test method".

### (2-1) Preparation of test solution

The live vaccine was dissolved in purified water to result in 10^{5.0} EID₅₀/mL or more per mL to prepare a virus solution. According to the plots for the test indicated in Table 6, the sample and the phosphate buffer (10 mL each) were aliquoted and the virus solution (1 mL) was added. After the addition of the virus solution, the obtained test solutions were left at room temperature (25°C) for the time respectively prescribed for the plots of the test.

**[Table 6]**

| Name of plot for test | Treatment | Sensitization period |
|---|---|---|
| Control plot (carried out twice) | Mixing phosphate buffer with virus solution | Zero or 3 hours from addition of virus |
| Test plot | Mixing sample with virus solution | Three hours from addition of virus |

### (2-2) Inoculation to embryonated chicken eggs

After the completion of the sensitization, the test solutions of the respective plots for the test were filtered through a membrane filter (0.45 µm) and diluted in a 10-fold series. The diluted solutions (0.1 mL each) were inoculated to the allantoic cavity of 10-day-old embryonated eggs derived from chickens (Lohmann VALO). Five embryonated chicken eggs were used per diluted solution at a concentration. Controls (three eggs) were also prepared to which only phosphate buffer (0.1 mL) was inoculated. After the inoculation, the eggs were cultured at 37°C for 7 days. On day 7 after the inoculation, the eggs were opened and the state of embryos for each plot of the test was observed by using the control chicken embryo as a reference. The chicken embryo that died or was altered (insufficient growth, size reduction) was evaluated as "with infection", and the logarithm of the minimum dilution factor of the test solution which showed the infection inhibitory effect was calculated as the viral titer (Behrens-Karber method) .

On the basis of the above results, the rate (%) of reduction of the test plot relative to the control plot at 3 hours after the start of the sensitization was calculated and the infection inhibitory effect was assessed. The rate of reduction was calculated according to the following equations: Logarithmic decrement = Log10(Viral titer of the control plot) - Log10(Viral titer of the test plot) Rate (%) of reduction (1 - 1/10Logarithmic decrement) × 100

### (3) Results

The results are shown in Table 7. In the control plot, a natural attenuation was observed between the start of the sensitization and three hours thereafter, and the viral titer shifted from approximately 10^{5.9} EID₅₀/mL to 10^{5.3} EID₅₀/mL. Meanwhile, in the test plot, the result was 10^{3.1} EID₅₀/mL at three hours after the start of the sensitization (rate of reduction: 99.37%). From the above results, it was found that the thermophile fermentation which is the sample obtained in "Test 1" has such a high inactivation effect as 99.37% in the reaction of 180 minutes against avian infectious bronchitis virus.

**[Table 7]**

| Name of plot for test | Viral titer at the start of the test | Viral titer at three hours after the start of the sensitization | Rate of reduction (%) |
|---|---|---|---|
| Control plot | 10^{5.9} | 10^{5.3} | Natural attenuation |
| Test plot | 10^{5.9} | 10^{3.1} | 99.37 |

### <Test 7: Test for virus (RNA virus) infection inhibitory effect-6>

The RNA virus infection inhibitory effect was studied in the same manner as in "Test 2" by using porcine epidemic diarrhea virus (PEDV) P-5V strain, which is a pig infectious coronavirus. In the present test, how the form (cell culture solution or culture supernatant of the cell culture solution) of thermophile "BP-03693" (including "BP-863") affects the effect.

Samples were prepared as follows:
- Sample 1: Cell culture solution (test solution of thermophile "BP-03693" prepared in the same manner as "Test 2")
- Sample 2: Sample containing live cells (vegetative cells) from the cell culture solution
- Sample 3: Sample containing vegetative cells of sporulated cells reduced by approximately 1/100 from the cell culture solution
- Sample 4: Culture supernatant of the cell culture solution (only supernatant recovered after centrifugation/precipitation (14000 rpm, 10 min) of cells from the test solution prepared in "Test 2")

The results are shown in Table 8. In the "sample 1", a natural attenuation of the viral load was observed between the start of the test and three hours thereafter (sample 1 (first round): 10^{6.3} to 10^{5.7} TCID₅₀/mL, sample 1 (second round): 10^{6.7} to 10^{6.1} TCID₅₀/mL). In the "sample 2", the result was 10^{2.9} TCID⁵⁰/mL between the start of the test and three hours thereafter (rate of reduction: 99.93%). In the "sample 3", the result was 10^{3.1} TCID₅₀/mL between the start of the test and three hours thereafter (rate of reduction: 99.74%). In the "sample 4", the result was 10^{1.7} TCID₅₀/mL between the start of the test and three hours thereafter (rate of reduction: 99.99%). From the above results, it was suggested that the fermentation metabolites released outside of cells rather than the presence of the cells were important for the virus inactivation effect.

**[Table 8]**

| Name of plot for test | Viral titer at the start of the test | Viral titer at three hours after the start of the sensitization | Rate of reduction (%) |
|---|---|---|---|
| Sample 1 (first round) | 10^{6.3} | 10^{5.7} | Natural attenuation |
| Sample 1 (second round) | 10^{6.7} | 10^{6.1} | Natural attenuation |
| Sample 2 | 10^{6.7} | 10^{2.9} | 99.93 |
| Sample 3 | 10^{6.3} | 10^{3.1} | 99.74 |
| Sample 4 | 10^{6.7} | 10^{1.7} | 99.99 |

### <Test 7: Test for virus (DNA virus) infection inhibitory effect>

A preliminary test was carried out by using hepatitis B virus (HBV) as a DNA virus. As a result, the infection inhibitor effect was observed in the presence of "PTA-1773", "BP-03693", "BP-1051" or "BP-863" or a sterilized solution thereof similarly to the RNA virus described above.

It was also observed that the abundance of HBV tended to decrease by approximately 10³ cells within 30 minutes of exposure of HBV to "PTA-1773", "BP-03693", "BP-1051" or "BP-863" or a sterilized solution thereof (filtration sterilization with 0.22 micrometers).

From the above results, the agent of the present invention may control not only RNA viruses but also DNA viruses by the action of extracellular metabolites derived from the cells.

A plant infectious DNA virus, Tomato yellow leaf curl virus (TYLCV), is known and is known to be mediated by whitefly. Application of a fermentation product containing "PTA-1773", "BP-03693", "BP-1051" or "BP-863" is found to tend to prevent the occurrence of Tomato yellow leaf curl in agricultural sites even though there is not always an adverse effect on whitefly. Root-knot nematodes mediate plant infectious RNA viruses, nepovirus and Tobravirus. Meanwhile, it is found that when a fermentation product containing "PTA-1773", "BP-03693", "BP-1051" or "BP-863" is applied to the soil abundant with root-knot nematodes, there is less crop damage even though the abundance of root-knot nematodes is not decreased. These phenomena on plants show that the agent of the present application may also exhibit the virus infection inhibitory effect on wide range of plant infectious DNA and RNA viruses. From these results, the agent of the present application may exhibit a direct virus infection inhibitory effect on, for example, plant infectious DNA viruses such as TYLCV (Geminiviridae, Begomovirus) and Tomato mosaic virus (Virgaviridae, Tobamovirus) and plant infectious RNA viruses such as nepovirus and Tobravirus without adversely affecting symbiotic organisms.

### <Industrial applicability of the present invention>

In the Hygiene Hypothesis (Strachan DP., Hayfever, hygiene, and household size. BMJ 299:1259-1260, 1989) discussed in the section of the Background Art points out concerns on extreme hygiene control. Specifically, it is suggested that the degree of the hygienic environment in early childhood may alter the prevalence of allergic diseases in future and an unhygienic environment may decrease onset of allergies in later stages. This hypothesis is now widely recognized, lots of data supporting this hypothesis have been accumulated and a relationship with other diseases in relation to formation of the intestinal microflora has also been pointed out. Namely, it is suggested that it is important for survival of animals to construct the environment that allows symbiotic relationship with environmental microorganisms.

Meanwhile, the form and extent of the symbiosis are open for discussion and it is difficult to provide a strict measure. The fundamental technique of the present application is known to have wide positive effects on animals and plants and various ecosystems such as stimulation of the immune system, reduction in lipid accumulation, plant growth promotion and suppression of production of greenhouse gases which put burdens on the environment. Enabling virus infection inhibition in addition to this foundation offers completely different perspectives from conventional virus infection inhibitors.

For example, bacteriophages of Cystoviridae which are also RNA viruses may infect soil symbiotic microorganisms, actinomycetes, involved in plant growth. Therefore, a solution containing "BP-863" or "BP-03693" may suppress death of actinomycetes and contribute to plant growth. Bacteriophages of Cystoviridae which are also RNA viruses may also infect Lactococcus also known as a probiotic in fish. Therefore, a solution containing "BP-863" or "BP-03693" may suppress infectivity of bacteriophages of Cystoviridae which are also RNA viruses and suppress death of Lactococcus, thereby increasing production of functional molecules derived from Lactococcus. The above is consistent with the fact that when the fish intestinal microflora was actually evaluated for salt water fish and fresh water fish administered with a fermented feed containing "BP-863" or "BP-03693", a tendency of an increase in Lactococcus was observed.

In addition, insects such as nematodes have been reported to mediate transmission of plant pathogenic viruses and the like. When these viruses are RNA viruses, the product of the present invention may reduce adverse effects on plants. It is expected that 2-aminoisobutyric acid (α-aminoisobutyric acid, hereinafter referred to as "Aib") and a complex thereof may be involved in the action mechanisms thereof. Aib is often called an unusual amino acid and is not a constituent of usual proteins unlike usual amino acids. From the characteristics thereof, Aib is known to be a constituent of lantibiotics that play roles similar to antibiotics. Research results in synthetic chemistry suggest that Aib forms a complex by papain (Tsuchiya, K. & Numata, K. Chemoenzymatic synthesis of polypeptides containing the unnatural amino acid 2-aminoisobutyric acid. Chem Commun (Camb) 53, 7318-7321, doi: 10.1039/c7cc03095a (2017)), which may be converted to a highly cell-penetrating complex (Terada, K. et al. Artificial Cell-Penetrating Peptide Containing Periodic alpha-Aminoisobutyric Acid with Long-Term Internalization Efficiency in Human and Plant Cells. ACS Biomater Sci Eng 6, 3287-3298, doi: 10.1021/acsbiomaterials.0c00182 (2020)).

Meanwhile, "BP-863" included in "BP-03693", namely Caldibacillus hisashii, and Paenibacillus No. 36 strain included in "BP-03693" contain papain analogous enzymes putative papain-like cysteine peptidase (SEQ ID NO: 1) and putative papain-like cysteine peptidase (DUF1796) (SEQ ID NO: 2), respectively. These enzymes are expected to be involved in synthesis of lantibiotics containing Aib as a constituent. Sequence information of these strains has been identified as a result of extraction of genomic DNAs and whole genome analysis thereof (Illumina HiSeq). From genomic genes of "BP-863", a gene (SEQ ID NO: 3) closely related to lantibiotic-related gene (sunA: lantibiotic anti peptide) of Bacillus thermoamylovorans has been detected. These are expected to mutually act to exhibit the effect on virus infection inhibition.

**[Table 9]**

| Papain-related gene of BP-863 putative papain-like cysteine peptidase (76% identity) |
|---|
| TTATTTCATTAAGGAAACACCTTGGAAAATTTTTGCCCAATATGGATCATTTTCAT CCCAAATTTCTTTATTTGGCAGTTCAACAAGAGTTACTGAGGAAAGCGGATACGGT TTTTCAATCATATGACTTATGTTTTTATGGTGGACTAATAGAATGTGAAAATCTCC GGCTACCATATTGGACAAAATCGTCTCCAATTCTTTTACATCTTCAAATTTTGCTT CATTTGCTTCAGTTCGGATGAATAAAATTCGTTTACTCGTATTCATTTTTTCAAGA AACCGATTAATTCTTCTGTCATATTTTGCTTTTACTTCCGCATACCCGCCTAAATG AGTTAATGTGTTTTTATCGGTTTTAAAATCATGGTTAAAGCCAATATCATAAACAA AGTCATGAACATATAAATCTGTAGGCGAGAGATAGCGATCGGGTTTTAAATGATTG ATATTCAAAAAATCGGAAAAACGGTTTTTAATCAGCAAATTGACTTTTGATAATTG TGGGGAACCAACCCAATCCAGCACACCCGAAAACGGACGTAACTGAAACTTCCGCA ATTGCATGGCAGCTAAACAAAGATGCCCTAAACTGAAAATGGAATCATACGTACCC TTTAATTTATGCAA (SEQ ID NO: 1) |

**[Table 10]**

| Papain-related gene of Paenibacillus No. 36 strain included in BP-03693 |
|---|
| Putative papain-like cysteine peptidase (DUF1796) (identity 77%) |
| TCACCTCAGGATAAGCGGCAAGGTCGGCGACCGTATTTTGCCCGCTTCGAAaTCGT GGCTGGAAaTGATGGAATTGGCGTCATCCGTCACCCAGATCATTCCGGTTTCCGTT ACCCCGCCTACCCGTAAATTTTCCAGCTCCATAAaTCCTTTGAACCTGGTGTCGAT CAGCCGGCAGACATCGTCAAGATTCGGCGAAGCCACCCAGTCGAaTACGCCGGCGT AGGGACGGAGATTGTTTTtCTCGAGCTGCAGCGAAGACAAACATAGGTCTCCCAAG CTGAAAaTCGCATCGTAGCCGCCTTTtAAaTCTGCCAGTTTCAT (SEQ ID NO: 2) |

**[Table 11]**

| LANTIBIOTIC-RELATED GENE OF BP-863 sunA; lantibiotic anti peptide (identity 96%) |
|---|
| GATGGCTAAGTTTGATGATTTCGATTTGGACATCGTTGTCAAAAAACAAGATACCA CTGTACAACCGAATGTTACAAGTAAATCTCTTTGTACGCCTGGTTGTATCACAGGT ATTTTAATGTGCTTAACTCAAAACTCCTGTGTATCTTGTAACTCTTGCATCAAATG TTAA (SEQ ID NO: 3) |

It is also expected that the environmental symbiotic function is improved by suppressing types and population of viruses in an environment in animals where symbiotic bacteria exist such as nose, oral cavity, skin, bronchus and intestine and in a natural environment where symbiotic bacteria exist such as soil, waste water, rivers, sea water and bottom sediment.

Upon constructing a sustainable society in future, the viewpoint of grasping symbiosis of the entire ecosystem is extremely important, and it is not sufficient to address only pathogens. The present application is expected to be an important technique from this viewpoint.

The virus infection inhibitor of the present invention may be used by slightly diluting the inhibitor when being applied to humans to minimize inhibition of symbiotic microorganisms. Because of the low environmental burden, the present invention may be used in various aspects. For example, the present invention is expected to be used for the purposes for which viral infection inhibition is sought without decreasing beneficial symbiotic bacteria such as applications to the soil, waste water, feed and the like for viral inhibition, application to the nose or skin, application to rooms and livestock housings and combined use with air purification systems.

## Claims

1. A virus infection inhibitor, comprising a microorganism of the genus Caldibacillus or a culture thereof.

2. The virus infection inhibitor according to claim 1, wherein the microorganism of the genus Caldibacillus contained is a microorganism having NITE Accession No. "BP-863".

3. The virus infection inhibitor according to claim 1, wherein the virus infection inhibitor is an environmentally symbiotic virus infection inhibitor comprising mixed microorganisms having a symbiotic function with humans, animals and plants and an ecosystem, and wherein
the mixed microorganisms are of the genus Caldibacillus and the genus Paenibacillus.

4. The environmentally symbiotic virus infection inhibitor according to claim 3, wherein the mixed microorganisms comprise a microorganism having NITE Accession No. "BP-03693".

5. The environmentally symbiotic virus infection inhibitor according to claim 4, wherein the mixed microorganisms comprising the microorganism having NITE Accession No. "BP-03693" comprise a microorganism having ATCC Accession No. "PTA-1773".

6. The virus infection inhibitor according to claim 5, wherein the mixed microorganisms having ATCC Accession No. "PTA-1773" comprise a microorganism having NITE Accession No. "BP-863" and a microorganism having NITE Accession No. "BP-1051".

7. The virus infection inhibitor according to any one of claims 1 to 6, wherein the virus is porcine epidemic diarrhea virus belonging to Alphacoronavirus, SARS-CoV-2 belonging to Betacoronavirus or avian infectious bronchitis virus (IB) belonging to Gammacoronavirus.

8. The virus infection inhibitor according to any one of claims 1 to 6, wherein the virus is seasonal influenza virus, avian influenza (H5N1) virus or swine fever virus (Flaviviridae, Pestivirus).

9. The virus infection inhibitor according to any one of claims 1 to 6 that does not have a negative impact on symbiotic organisms, wherein the virus is norovirus, feline calicivirus, norovirus, Norwalk virus or rabbit hemorrhagic virus all of which are of the family Caliciviridae, a plant infectious virus (begomovirus, tobamovirus, nepovirus, tobravirus), a bacteriophage (Cystoviridae) or a hepatitis virus.
